# FASCICULE DE BREVET EUROPEEN

(11) **EP 2 575 967 B1**
(45) Date de publication et mention de la délivrance du brevet: **20.04.2016**
(21) Numéro de dépôt: 11792002.5
(22) Date de dépôt: 07.06.2011
(51) Int. Cl.: A61N 7/02, B06B 1/06, G10K 11/32, G10K 11/34

(54) **TRANSDUCTEUR D'ULTRASONS À USAGE MÉDICAL**
ULTRASCHALLWANDLER FÜR MEDIZINISCHE ZWECKE
ULTRASOUND TRANSDUCER FOR MEDICAL USE

(30) Priorité: 07.06.2010 FR 1054454
(43) Date de publication de la demande: 10.04.2013
(73) Titulaire: Image Guided Therapy, 33600 Pessac (FR)
(72) Inventeur: AUBOIROUX, Vincent, F-74100 Annemasse (FR); DUMONT, Erik, F-33800 Bordeaux (FR); SALOMIR RARES, Vincent, F-74100 Ambilly (FR)
(74) Mandataire: Ipside
(86) Numéro de dépôt international: PCT/FR2011/051288
(87) Numéro de publication internationale: WO 2011/154654

(56) Documents cités:
- WO-A2-2008/015521
- US-A- 6 135 971

## Description

La présente invention concerne un transducteur d'ultrasons en réseau phasé à usage médical, notamment pour des applications thérapeutiques. Elle concerne également une machine de traitement thérapeutique équipée d'au moins un tel transducteur d'ultrasons.

On connaît des méthodes d'ablation non invasive de tissus cancéreux par utilisation d'ultrasons focalisés de haute intensité. Ces méthodes sont basées sur l'élévation localisée et à distance de la température des tissus biologiques afin de nécroser les tissus cancéreux sans toucher aux tissus environnants.

Cette élévation locale de température est obtenue en focalisant des ultrasons dans les tissus biologiques, l'appareil générant les ondes ultrasonores étant positionné à l'extérieur du corps humain.

Il est connu que l'efficacité thérapeutique du traitement est améliorée lorsque les ultrasons focalisés sont concentrés en un point focal de petit volume par rapport à un point focal diffus, sous réserve de couvrir la totalité du volume tumoral par déplacement de ce point focal.

Un dispositif de traitement efficace doit donc combiner un point focal de faible dimension et le moyen de déplacer ce point focal afin de couvrir l'intégralité du volume cible à traiter.

La focalisation d'ultrasons peut être obtenue à l'aide d'une grande variété de transducteurs, notamment en termes de forme et de matériaux constitutifs.

Un transducteur très largement répandu a ainsi une forme de calotte sphérique et est réalisé à partir d'une pièce de céramique piézoélectrique ou d'une structure piezocomposite mise en forme. L'intérêt d'un tel transducteur réside alors dans sa capacité à focaliser naturellement un faisceau ultrasonore au centre géométrique de la calotte sphérique.

Ce type de transducteur permet ainsi d'obtenir une forte concentration des ultrasons au point focal de manière à induire une élévation locale de température compatible avec un traitement thermique des tissus.

Toutefois, lorsque la cible thérapeutique est placée dans un organe mobile tel que le foie ou le rein, qui se déplace de quelques centimètres au cours du cycle respiratoire, le point focal doit non seulement pouvoir couvrir l'ensemble du volume cible mais également pouvoir accompagner, pour le moins partiellement, le mouvement de la cible.

Ce suivi partiel de la cible à traiter impose une contrainte forte sur l'amplitude de déplacement nécessaire au point focal pour traiter l'ensemble du volume cible.

Plusieurs solutions ont été proposées pour augmenter le volume couvert par les ultrasons focalisés.

La solution technique la plus simple consiste à fixer le transducteur d'ultrasons sur un système de positionnement motorisé de telle manière que la position et/ou l'orientation du transducteur puissent être modifiée à distance.

Cette technique offre une très grande flexibilité dans le déplacement du point focal. Elle autorise potentiellement un contrôle du déplacement du point focal de manière à suivre une cible dans un organe mobile tout au long du cycle respiratoire.

Cependant, cette solution présente des inconvénients majeurs lorsqu'elle est installée dans un dispositif d'imagerie par résonance magnétique (IRM).

Tout d'abord, l'encombrement mécanique est important dans l'espace restreint de l'aimant du dispositif IRM.

On observe également des perturbations de l'imagerie IRM liées à des interférences radiofréquences dues au système de motorisation et des perturbations des cartographies de température obtenues par imagerie de phase en IRM. Ces dernières résultent de variations de susceptibilité magnétique liées aux déplacements du transducteur dans le champ magnétique de l'aimant.

Or, un contrôle par imagerie du patient durant le traitement thérapeutique est essentiel pour garantir la sécurité du patient et assurer la fiabilité de la procédure. L'imagerie IRM présente deux avantages majeurs en terme de contrôle d'ablation thermique par rapport aux autres techniques d'imagerie disponibles telles que l'échographie.

Tout d'abord, l'imagerie IRM permet de bien visualiser la tumeur et les tissus sensibles environnants, ce qui est indispensable pour une planification optimale du traitement thérapeutiques. Ensuite, l'IRM fournit une cartographie de température fiable dans l'ensemble du volume cible pendant le traitement, ce qui permet non seulement de visualiser l'ablation thermique en temps réel mais surtout d'adapter le traitement en fonction de la réponse des tissus.

Dans le document "IEEE Transactions on ultrasonics, ferroelectrics and frequency control", Vol. 36, N° 5. Septembre 1989, EBBINI et al. introduisent la notion de réseau phasé dans lequel le transducteur d'ultrasons est divisé en plusieurs éléments actifs, chacun de ces éléments actifs étant excité par un signal d'excitation dont la phase est contrôlée individuellement.

La position du point focal peut alors être modifiée en appliquant une loi de phase appropriée aux éléments composant le transducteur. Ce procédé est couramment appelé "beamforming" (mise en forme de faisceau), ou encore déflexion électronique du point focal, et s'applique aux réseaux dits phasés. La formation d'un volume de couverture, encore appelé zone de focalisation accessible, par déflexion électronique du point de focalisation d'une matrice d'éléments transducteur est ainsi divulguée. Ce volume de couverture se caractérise par une énergie acoustique du point focal défléchi d'au moins 50% de l'énergie du point focal au point de convergence géométrique, lequel est le centre de la calotte sphérique.

Ce document enseigne également comment générer des "patterns" de focalisation dans le volume de couverture. Ces "patterns" peuvent, par exemple, être des points focaux multiples. En résumé, toutes les utilisations d'un transducteur en réseau phasé (déflection et multi-focus) sont décrites dans ce document de l'état de l'art.

A titre illustratif, une telle mise en forme du faisceau ultrasonore, permet d'obtenir une focalisation à partir d'une surface support plane et autorise le déplacement du point focal dans une petite zone sans déplacement mécanique du transducteur, et avec un temps de réponse beaucoup plus court qu'avec un système de déplacement motorisé classique.

Cependant, des problèmes apparaissent lorsque le découpage du transducteur en différents éléments actifs est trop régulier. Des points focaux secondaires apparaissent en fonction de la position du point focal primaire. Ces points focaux secondaires, encore appelés "grating lobes" (lobes secondaires), peuvent provoquer des élévations de température en des points non désirés et nuisent ainsi à l'utilisation de transducteurs en réseau phasé pour l'ablation thermique.

Des progrès ont été apportés avec l'apparition de transducteurs dont les éléments sont repartis pseudo aléatoirement sur la surface support. Cette distribution pseudo-aléatoire limite l'apparition d'éventuels points chauds non désirés lors de la déflection électronique du faisceau, ce qui explique qu'il s'agisse du type de transducteur le plus couramment utilisé aujourd'hui.

La principale limitation ce type de transducteur à ultrasons reste la taille relativement faible de la zone de couverture du point focal.

La déflection électronique est principalement limitée par le diagramme de rayonnement des émetteurs individuels (inversement proportionnel au produit diamètre x fréquence) et dépend de la géométrie du transducteur, en particulier le rayon de courbure de la calotte sphérique. Sachant qu'à la fréquence de travail le faisceau émis est directif, une tentative de focalisation loin du point focal naturel se traduira par une forte baisse d'efficacité.

Typiquement, pour un transducteur de 13cm de rayon de courbure, fonctionnant à une fréquence de 1MHz, avec 256 éléments circulaires de 6mm de diamètre, la déflection maximale du point focal est d'environ 15 mm par rapport au point focal naturel (centre de la calotte sphérique).

Cette couverture est compatible avec les méthodes de compensation de petits déplacements des organes afin de créer une lésion précise, sans effet de flou induit par le mouvement, ou de créer une lésion plus large dans le cas d'organe immobile.

Cependant, cette méthode ne permet pas de traiter un large volume en présence de mouvement respiratoire sans avoir recours à un système de déplacement mécanique.

Les transducteurs en réseau phasé, aux éléments dispersés de manière aléatoire sur une surface concave, sont en général peu denses. En effet, si la surface des éléments est trop importante par rapport à la surface du transducteur (> 50%) la position des éléments devient moins aléatoire et à la limite d'un transducteur compact ou la surface des éléments approche 70% de la surface du transducteur, la répartition des éléments devient totalement ordonnée avec pour conséquence, l'apparition de points focaux ou lobes secondaires.

La présente invention vise à pallier ces divers inconvénients en proposant un transducteur d'ultrasons particulièrement simple dans sa conception et dans son mode opératoire, économique et permettant l'obtention d'une zone de couverture plus large qu'avec les techniques connues de l'état de l'art.

A cet effet, l'invention concerne un transducteur d'ultrasons en réseau phasé à usage médical, ce transducteur comprenant un support unique et un ensemble d'éléments transducteurs distribués de manière aléatoire à la surface dudit support, lesdits éléments transducteurs étant fixes.

Selon l'invention,
- cet ensemble comporte au moins deux sous-ensembles d'éléments transducteurs, chacun desdits sous-ensembles d'éléments transducteurs ayant un point de convergence géométrique distinct de manière à focaliser une énergie ultrasonore dans un volume de couverture distinct de sorte que l'arrangement desdits volumes de couverture couvre un volume cible à traiter, et
- des moyens de pilotage pour piloter lesdits éléments transducteurs indépendamment les uns des autres.

Ce transducteur d'ultrasons permet avantageusement de définir au moins deux volumes de couverture distincts, lesquels peuvent être contigus, voire présenter un volume de recoupement. On peut ainsi s'assurer du traitement d'un volume cible mobile entre deux points extrêmes, la plage de déplacement de ce volume cible étant couverte par des volumes de couverture distincts.

Un des intérêts de ce transducteur d'ultrasons est, par conséquent, d'augmenter la précision du traitement thérapeutique, en délivrant la dose thérapeutique à l'intérieur d'une zone de traitement couvrant le mouvement d'un volume cible sans déplacement mécanique du transducteur. On évite ainsi les artefacts dynamiques de susceptibilité en imagerie IRM.

Ce transducteur d'ultrasons permet également de traiter en une seule fois des volumes de tissus cancéreux plus importants pour une cible fixe.

Ce transducteur permet encore d'améliorer la prédiction de la dose réellement déposée sur les tissus biologiques puisqu'il est possible de s'assurer d'un traitement effectif continu dans le volume cible à traiter.

Les moyens de pilotage émettent des signaux d'excitation ayant des fréquences d'excitation comprises entre 0,5 MHz et 10 MHz. De préférence, la fréquence d'excitation de chaque sous-ensemble d'éléments transducteurs est comprise entre 0,5 MHz et 3 MHz, et encore mieux entre 1 et 2 MHz pour des applications thérapeutiques.

On entend par "éléments transducteurs distribués de manière aléatoire", une distribution aléatoire ou quasi-aléatoire des éléments transducteurs, la position de certains de ces éléments transducteurs ayant pu être modifiée à partir d'une position purement aléatoire de manière à conserver un espace minimal entre les éléments transducteurs. Cette distance minimale est, par exemple, de l'ordre de 0,5 mm prise entre les bords des éléments transducteurs. On obtient donc une distribution semi aléatoire dispersée des éléments transducteurs de chaque sous-ensemble d'éléments transducteurs, l'ensemble des éléments transducteurs à la surface du support unique étant entremêlé, la distribution ainsi obtenue étant avantageusement compacte.

On définit le "volume de couverture" d'un sous-ensemble d'éléments transducteurs comme étant le volume couvert par déflection électronique du point focal généré par ce sous-ensemble autour de son point focal naturel. Le "point focal naturel" est défini comme le point de convergence géométrique du sous-ensemble d'éléments transducteurs, le point de convergence d'un sous-ensemble étant lui-même défini comme le point vers lequel sont orientés les vecteurs normaux à la surface de chacun des éléments transducteurs constituant ce sous-ensemble.

De préférence, le volume de couverture est la zone autour du point focal naturel du sous-ensemble où le point focal obtenu par déflection électronique a au moins 50% de la puissance acoustique du point focal naturel du sous-ensemble d'éléments transducteurs.

Bien entendu, il ne faut pas confondre ici "point de convergence géométrique" (ou point focal naturel) d'un sous-ensemble d'éléments transducteurs avec des points focaux multiples que l'on peut obtenir par l'action d'un réseau phasé.

On entend par " volume cible à traiter", le volume de la cible à traiter celle-ci étant fixe ou le volume total généré par une cible mobile ayant un volume à traiter V.

Le nombre d'éléments transducteurs de chaque sous-ensemble est déterminé pour apporter une énergie suffisante pour assurer la nécrose des tissus biologiques dans le volume de couverture généré par ce sous-ensemble d'éléments transducteurs. Par ailleurs, on entend par "les éléments transducteurs étant fixes" que ces éléments transducteurs individuels sont fixes en position et en orientation à la surface du support unique.

Dans différents modes de réalisation particuliers de ce transducteur d'ultrasons, chacun ayant ses avantages particuliers et susceptibles de nombreuses combinaisons techniques possibles:
- les éléments transducteurs de chaque sous-ensemble sont placés à la surface dudit support unique de sorte que les points de convergence de ces sous-ensembles sont alignés selon au moins un axe.
De préférence, le volume cible à traiter étant mobile le long d'un axe de déplacement, lesdits points de convergence sont placés sur cet axe de déplacement.
- ledit ensemble comprenant au moins quatre sous-ensembles, les éléments transducteurs de chaque sous-ensemble sont placés à la surface dudit support unique de sorte que les points de convergence d'au moins deux premiers sous-ensembles sont placés sur un premier axe et les points de convergence d'au moins deux seconds sous-ensembles sont placés sur un second axe, lesdits premier et second axes étant non parallèles,
Avantageusement, ces axes sont perpendiculaires.

Alternativement, les points de convergence des sous-ensembles du transducteur d'ultrasons étant placés le long d'un seul axe, le support du transducteur peut être monté sur une platine mobile en rotation de manière à déplacer l'orientation de cet axe d'au moins 90°, et encore mieux 180°.
- chacun desdits sous ensembles d'éléments transducteurs ayant une fréquence de résonance propre et distincte, un élément transducteur d'un sous-ensemble est relié, ou encore câblé, à un élément transducteur de chaque autre sous-ensemble du transducteur d'ultrasons en réseau phasé de l'invention, de sorte que le choix d'une fréquence d'excitation donnée délivrée par les moyens de pilotage, sélectionne et active un unique sous-ensemble d'éléments transducteurs.
De préférence, on reliera, ou encore câblera, en parallèle des éléments transducteurs appartenant à des sous-ensembles différents, les plus proches. Il est ainsi possible de diviser le nombre de câbles nécessaires en sortie du support unique pour alimenter les sous-ensembles d'éléments transducteurs par N, où N est un nombre entier positif représentant le nombre de sous-ensembles d'éléments transducteurs du transducteur d'ultrasons en réseau phasé de l'invention.
A titre purement illustratif, pour un transducteur d'ultrasons comprenant 2 sous-ensembles de 128 éléments transducteurs chacun, soit 256 éléments transducteurs sur le support unique, le nombre de câbles nécessaires pour alimenter ces éléments transducteurs depuis les moyens de pilotage est de 128.

Alternativement, les moyens de pilotage émettent un signal d'excitation principal comprenant une superposition d'au moins deux signaux d'excitation secondaires ayant des fréquences d'excitation distinctes, chacun desdits sous-ensembles d'éléments transducteurs répondant à une seule fréquence d'excitation, ces fréquences d'excitation étant différentes pour tous les sous-ensembles,

De préférence, le signal d'excitation principal étant un signal multispectral, les signaux d'excitation secondaires sont des signaux d'excitation monochromatiques.
- les moyens de pilotage sont reliés à chaque élément transducteur individuellement.
Alternativement, les moyens de pilotage étant reliés auxdits éléments transducteurs par des liaisons, au moins certaines desdites liaisons relient lesdits moyens de pilotage à des groupes d'éléments transducteurs, chaque groupe comprenant au moins un élément transducteur d'au moins deux sous-ensembles distincts.
Les éléments transducteurs appartenant à chacun desdits groupes sont ainsi électriquement connectés en parallèle.

L'invention vise également un procédé de fabrication d'un transducteur d'ultrasons à usage médical tel que décrit précédemment.

Selon l'invention, ce procédé comporte au moins les étapes successives suivantes, lesdites étapes étant réalisées par au moins une unité de calcul :
a) ayant un unique support, on répartit de manière aléatoire à la surface dudit support, des éléments transducteurs définissant un premier sous-ensemble d'éléments transducteurs destiné à focaliser une énergie ultrasonore dans un premier volume de couverture,
b) les éléments transducteurs du premier sous-ensemble étant fixes, on disperse à la surface dudit support comprenant ledit premier sous-ensemble, des éléments transducteurs définissant un deuxième sous-ensemble d'éléments transducteurs destiné à focaliser une énergie ultrasonore dans un deuxième volume de couverture distinct dudit premier volume,
c) on ajuste la position de chacun des éléments transducteurs de ce deuxième sous-ensemble de manière à limiter les lobes de réseaux qui pourraient apparaître dans le plan focal si ce sous-ensemble d'éléments transducteurs comportait des symétries, tout en conservant une distance minimale d entre chaque élément transducteur,
d) les éléments transducteurs de chaque sous-ensemble ainsi formé étant fixes, on répète éventuellement les étapes b) et c) pour chaque nouveau sous-ensemble d'éléments transducteurs de ce transducteur destiné à focaliser une énergie ultrasonore dans un volume de couverture distinct desdits autres volumes de couverture.

Après optimisation par calcul de la position des éléments transducteurs à la surface du support unique, on réalise le support unique du transducteur par exemple par prototypage rapide. A titre purement illustratif, le transducteur peut alors être réalisé par frittage laser de poudres ou encore par stéréo lithographie. Au droit de la position des éléments transducteurs des sous-ensembles, ce support unique peut comporter des canaux débouchant pour le passage des câbles.

Le support unique du transducteur peut ainsi être d'un seul tenant en matière plastique, ce qui le rend avantageusement compatible avec l'imagerie par résonance magnétique (IRM). Les éléments transducteurs qui peuvent être des céramiques piézo-électriques d'un seul tenant ou un assemblage de plusieurs émetteurs ultrasonores individuels, sont ensuite rapportés et fixés sur le support unique.

Avantageusement, la détermination du point de convergence géométrique de chaque sous-ensemble d'éléments transducteurs étant déterminée par l'orientation du vecteur normal de chacun des éléments transducteurs, on s'affranchit d'une forme particulière de support unique contrairement aux transducteurs de l'art antérieur. Le support unique de l'invention n'est donc pas forcément une calotte sphérique mais peut avantageusement être adapté à l'anatomie de l'organe cible. A titre d'exemple non limitatif, pour traiter un rein, le support unique peut prendre une forme allongée et incurvée. Alternativement, pour traiter un sein, le support unique peut prendre une forme cylindrique ou encore de cône tronqué assurant le recouvrement intégral de l'organe à traiter.

A titre purement illustratif, l'unité de calcul est un ordinateur comportant des moyens d'affichage.

L'étape c) permet de prendre en compte les contraintes de placement des éléments transducteurs d'un nouveau sous-ensemble par rapport aux éléments transducteurs déjà fixés en position à la surface du support unique. Le placement des éléments transducteurs est réalisé de manière aléatoire.

Les éléments transducteurs des différents sous-ensembles sont donc distribués aléatoirement en étant mélangés à la surface du support unique.

L'invention vise encore une machine de traitement thérapeutique équipée d'au moins un transducteur d'ultrasons à usage médical tel que décrit précédemment.

Cette machine de traitement thérapeutique peut comporter un dispositif d'imagerie médicale tel qu'un dispositif d'imagerie par résonance magnétique (IRM) afin de suivre en temps réel l'évolution du traitement thérapeutique opéré par le transducteur d'ultrasons.

Alternativement, le dispositif d'imagerie peut être un dispositif d'imagerie par tomographie ou encore un dispositif d'imagerie par échographie.

Le dispositif d'imagerie par tomographie comprend une source de rayons X.

L'invention sera décrite plus en détail en référence aux dessins annexés dans lesquels:
- la figure 1 est une représentation schématique d'un transducteur d'ultrasons à usage médical selon un mode de réalisation préféré de l'invention ;
- la figure 2 est une vue en coupe selon l'axe A-A du transducteur de la Figure 1 ;
- la figure 3 est une vue de dessus du transducteur de la Figure 1 ;
- la figure 4 est une autre vue en coupe selon l'axe A-A du transducteur de la Figure 1 ;
- la figue 5 est une vue élargie de la Figure 4 dans laquelle deux éléments transducteurs appartenant à des sous-ensembles distincts sont connectés en parallèle;

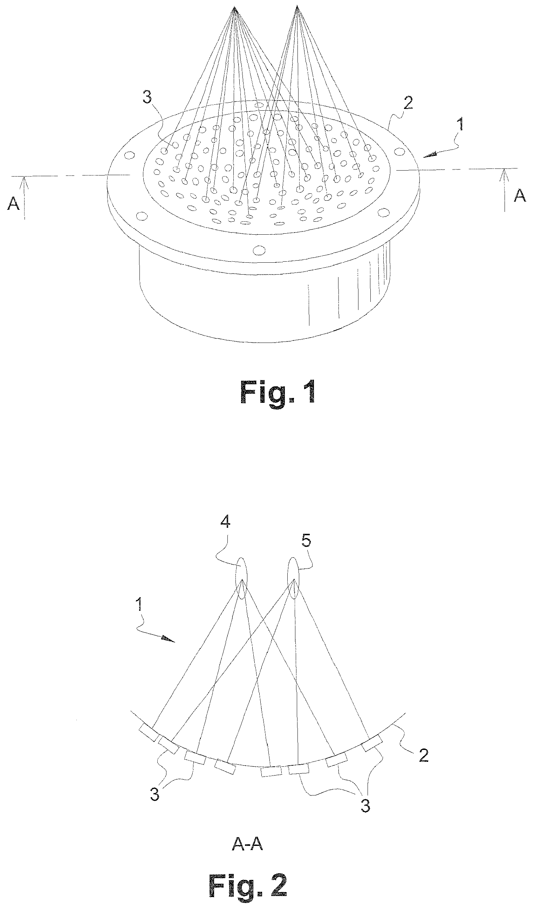

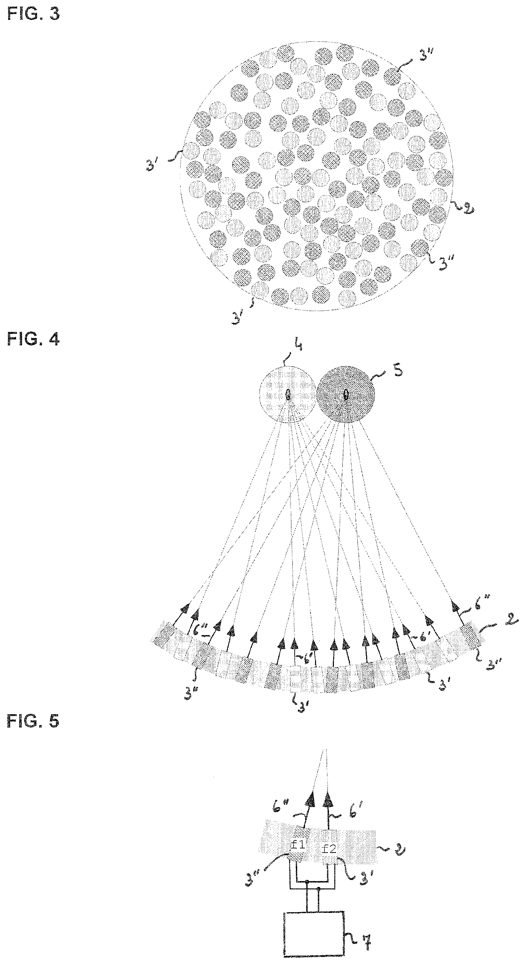

Les Figures 1 à 5 montrent un transducteur d'ultrasons à usage médical selon un mode de réalisation préféré de l'invention. Ce transducteur d'ultrasons 1 comprend un seul support 2, appelé par la suite matrice. Cette matrice 2 a ici la forme d'une calotte sphérique. A titre purement illustratif, cette matrice 2 qui est en matière plastique, a un rayon de courbure de 12 cm.

La matrice 2 est percée d'orifices cylindriques (non représentés) distribués aléatoirement à la surface de la matrice 2 et orientés de manière à ce que les axes de ces orifices cylindriques pointent ici vers deux points de convergence distincts.

Le transducteur d'ultrasons comprend également des émetteurs ultrasonores 3 qui sont fixés dans cette matrice au niveau de ces orifices cylindriques de manière à définir deux sous-ensembles d'émetteurs.

Ces deux sous-ensembles sont destinés à focaliser une énergie ultrasonore dans des volumes de couverture distincts de sorte que l'arrangement de ces deux volumes de couverture couvre un volume cible à traiter.

Chaque émetteur ultrasonore 3 est ici une céramique piézo-électrique monobloc reçue dans un cylindre rigide, ce cylindre comprenant également des composants passif pour l'adaptation d'impédance et la connectique permettant de relier l'émetteur ultrasonore 3 à des moyens de pilotage (non représentés) de ces émetteurs tels qu'un générateur de signaux d'excitation.

Selon un mode de mise en oeuvre de l'invention, les émetteurs ultrasonores 3 sont des éléments piézoélectriques commercialisés par la société Ferroperm Piezoceramics A/S, Kvistgaard, Danemark. Ils ont les caractéristiques suivantes :
◆ Premier sous-ensemble d'émetteurs ultrasonores 3 :
   ∘ épaisseur : 2 mm
   ∘ fréquence de résonance : 1,03 MHz
   ∘ diamètre : 5 mm
   ∘ matériau : PZ26
◆ Second sous-ensemble d'émetteurs ultrasonores 3 :
   ∘ épaisseur : 2,22 mm
   ∘ fréquence de résonance : 0,96 MHz
   ∘ diamètre : 5 mm
   ∘ matériau : PZ26

Dans le mode de réalisation le plus simple décrit ci-dessus, deux points de convergence sont définis et deux sous-ensembles de 64 orifices cylindriques sont réalisés dans la matrice 2, de manière à accueillir 128 émetteurs ultrasonores 3 pointant vers deux points focaux 4, 5 naturels distincts séparés d'environ 2,5 cm.

Les deux points focaux 4, 5 naturels sont orientés suivant la direction de mouvement principal d'un organe cible à traiter (non représenté). Suivant la position de la cible à un moment donné, le sous-ensemble d'émetteurs ultrasonores 3 offrant la meilleure capacité de focalisation sur cette cible est activé avec une loi de phase adaptée, c'est-à-dire que cette loi tient compte de la position de la cible par rapport aux émetteurs ultrasonores individuels. La couverture globale du transducteur (sans déplacement mécanique) est donc la superposition des volumes de couverture générés par les deux sous-ensembles, chaque sous-ensemble d'émetteurs ultrasonores couvrant approximativement un volume de couverture de forme sphérique qui est ici de 3 cm de diamètre.

Alternativement, les émetteurs sonores 3 des deux sous-ensembles ont des fréquences de résonance légèrement différentes, de telle manière que les signaux à la fréquence de résonance d'un sous-ensemble n'affectent pas les émetteurs ultrasonores 3 de l'autre sous-ensemble. Ainsi, il est possible de câbler des paires d'émetteurs ultrasonores, un de chaque sous-ensemble, en parallèle.

Le nombre de liaisons, ou câbles, nécessaires est ainsi divisé par deux, soit 64 câbles pour connecter les 128 émetteurs ultrasonores du transducteur de la Figure 1. La sélection d'un des deux sous-ensembles est réalisée par le choix de la fréquence du signal envoyé qui correspond à la fréquence de résonance d'un des deux sous-ensembles.

En utilisant le multiplexage spectral, le nombre de voies de génération nécessaires à l'alimentation d'un transducteur d'ultrasons comprenant plusieurs sous-ensembles (respectivement associés à différentes zones de la tumeur) est divisé par un nombre entier. Les points focaux crées par les sous-ensembles d'émetteurs ultrasonores 3 peuvent être activés soit de manière séquentielle, soit en parallèle.

L'activation en parallèle est rendue possible par l'utilisation d'un générateur de signaux délivrant des signaux multi fréquentiels. Ces signaux d'excitation sont par exemple la somme de plusieurs signaux purement sinusoïdaux, ayant chacun leur loi de phase propre.

On décrira par la suite un procédé de fabrication d'un transducteur d'ultrasons selon les figures 1 à 5, dans lequel
a) on positionne à la surface du support unique n éléments transducteurs 3', 3", n étant ici égal à 128 à titre purement illustratif,
b) on affecte de manière aléatoire chaque élément transducteur 3', 3" ainsi placé à un sous-ensemble d'éléments transducteurs (cercle plein, cercle hachuré), le nombre de sous-ensembles étant donné par le nombre de points de convergence géométrique 4, 5 souhaités,
   Ce nombre étant ici égal à 2, on attribue de manière aléatoire chaque élément transducteur 3', 3" à un des deux sous-ensembles, soit 64 éléments par sous-ensemble d'éléments transducteurs.
c) on oriente le vecteur normal 6', 6" de chacun desdits éléments transducteurs 3', 3" en fonction du point de convergence géométrique 4, 5 du sous-ensemble auquel a été affecté ledit élément transducteur 3',3",
d) on connecte les éléments transducteurs 3', 3" à des moyens de pilotage 7 pour piloter ces éléments transducteurs 3', 3" indépendamment les uns des autres.

On ajuste éventuellement l'affectation d'au moins certains éléments transducteurs 3', 3" pour obtenir une répartition homogène des sous-ensembles d'éléments transducteurs à la surface du support unique 2.

De préférence, à l'étape a), on place autant d'éléments transducteurs que nécessaire de sorte que les surfaces cumulées de l'ensemble de ces éléments transducteurs représentent au moins cinquante pour cent (50%) de la surface dudit support unique.

Alternativement, et dans le cadre d'un transducteur d'ultrasons comportant uniquement deux sous-ensembles d'éléments transducteurs, on peut fabriquer ce transducteur en répartissant de manière aléatoire les éléments transducteurs par paire.

Chaque sous-ensemble d'éléments transducteurs 3', 3" ayant une fréquence de résonance propre et distincte, f₁ et f₂, un élément transducteur 3' d'un premier sous-ensemble (cercle plein) est connecté en parallèle à un élément transducteur 3" du second sous-ensemble (cercle hachuré) de sorte que le choix d'une fréquence d'excitation donnée f₁ ou f₂ délivrée par les moyens de pilotage 7, permet de sélectionner un des deux sous-ensembles d'éléments transducteurs.

## Revendications

1. Transducteur d'ultrasons en réseau phasé à usage médical, ledit transducteur comprenant un support (2) unique et un ensemble d'éléments transducteurs (3) qui sont fixes à la surface dudit support (2), dans lequel
- ledit ensemble comporte au moins deux sous-ensembles d'éléments transducteurs (3), chacun desdits sous-ensembles d'éléments transducteurs (3) ayant un point de convergence géométrique distinct de manière à focaliser une énergie ultrasonore dans un volume de couverture (4, 5) distinct de sorte que l'arrangement desdits volumes de couverture couvre un volume cible à traiter,
- des moyens de pilotage pour piloter lesdits éléments transducteurs (3) indépendamment les uns des autres, et dans lequel
- les éléments transducteurs (3) des différents sous-ensembles sont distribués aléatoirement en étant mélangés les uns parmi les autres à la surface du support (2) unique.

2. Transducteur selon la revendication 1, **caractérisé en ce que** les éléments transducteurs (3) de chaque sous-ensemble sont placés à la surface dudit support (2) unique de sorte que les points de convergence de ces sous-ensembles sont alignés selon au moins un axe.

3. Transducteur selon la revendication 2, **caractérisé en ce que** le volume cible à traiter étant mobile le long d'un axe de déplacement, lesdits points de convergence sont placés sur ledit axe de déplacement.

4. Transducteur selon la revendication 2, **caractérisé en ce que** ledit ensemble comprenant au moins quatre sous-ensembles, les éléments transducteurs (3) de chaque sous-ensemble sont placés à la surface dudit support (2) unique de sorte que les points de convergence d'au moins deux premiers sous-ensembles sont placés sur un premier axe et les points de convergence d'au moins deux seconds sous-ensembles sont placés sur un second axe, lesdits premier et second axes étant non parallèles.

5. Transducteur selon l'une quelconque des revendications 1 à 4, **caractérisé en ce que** chacun desdits sous ensembles d'éléments transducteurs ayant une fréquence de résonance propre et distincte, un élément transducteur d'un sous-ensemble est relié à un élément transducteur de chaque autre sous-ensemble du transducteur d'ultrasons en réseau phasé de sorte que le choix d'une fréquence d'excitation donnée, délivrée par les moyens de pilotage, active un unique sous-ensemble d'éléments transducteurs.

6. Transducteur selon l'une quelconque des revendications 1 à 4, **caractérisé en ce que** lesdits moyens de pilotage émettent un signal d'excitation principal comprenant une superposition d'au moins deux signaux d'excitation secondaires ayant des fréquences d'excitation distinctes, chacun desdits sous-ensembles d'éléments transducteurs (3) répondant à une seule fréquence d'excitation, ces fréquences d'excitation étant différentes pour tous les sous-ensembles.

7. Transducteur selon l'une quelconque des revendications 1 à 6, **caractérisé en ce que** lesdits moyens de pilotage étant reliés auxdits éléments transducteurs (3) par des liaisons, au moins certaines desdites liaisons relient lesdits moyens de pilotage à des groupes d'éléments transducteurs (3), chaque groupe comprenant au moins un élément transducteur d'au moins deux sous-ensembles distincts.

8. Transducteur selon l'une quelconque des revendications 1 à 7, **caractérisé en ce que** les surfaces cumulées de l'ensemble des éléments transducteurs représentent au moins cinquante pour cent (50%) de la surface dudit support unique.

9. Transducteur selon l'une quelconque des revendications 1 à 8, **caractérisé en ce que** la géométrie dudit support unique est choisie dans le groupe comprenant cône tronqué, paraboloïde, cylindrique ou encore de forme allongée et incurvée.

10. Procédé de fabrication d'un transducteur d'ultrasons selon l'une quelconque des revendications 1 à 9, **caractérisé en ce qu'**il comporte au moins les étapes successives suivantes, lesdites étapes étant réalisées par au moins une unité de calcul :
a) ayant un unique support (2), on répartit de manière aléatoire à la surface dudit support (2), des éléments transducteurs (3) définissant un premier sous-ensemble d'éléments transducteurs (3) destiné à focaliser une énergie ultrasonore dans un premier volume de couverture (4, 5),
b) les éléments transducteurs (3) du premier sous-ensemble étant fixes, on disperse à la surface dudit support (2) comprenant ledit premier sous-ensemble, des éléments transducteurs (3) définissant un deuxième sous-ensemble d'éléments transducteurs (3) destiné à focaliser une énergie ultrasonore dans un deuxième volume de couverture (4, 5) distinct dudit premier volume,
c) on ajuste la position de chacun des éléments transducteurs (3) de ce deuxième sous-ensemble de manière à limiter les lobes de réseaux qui pourraient apparaître dans le plan focal si ce sous-ensemble d'éléments transducteurs (3) comportait des symétries, tout en conservant une distance minimale d entre chaque élément transducteur,
d) les éléments transducteurs (3) de chaque sous-ensemble ainsi formé étant fixes, on répète éventuellement les étapes b) et c) pour chaque nouveau sous-ensemble d'éléments transducteurs (3) destiné à focaliser une énergie ultrasonore dans un volume de couverture (4, 5) distinct desdits autres volumes de couverture, et
- l'ensemble des éléments transducteurs (3) à la surface du support (2) unique étant entremêlé.

11. Procédé de fabrication d'un transducteur d'ultrasons selon l'une quelconque des revendications 1 à 9, **caractérisé en ce qu'**il comporte au moins les étapes successives suivantes, certaines desdites étapes étant réalisées par au moins une unité de calcul :
a) on positionne à la surface d'un support unique n éléments transducteurs,
b) on affecte de manière aléatoire chaque élément transducteur ainsi placé à un sous-ensemble d'éléments transducteurs, le nombre de sous-ensembles étant donné par le nombre de points de convergence géométrique souhaités,
c) on oriente le vecteur normal de chacun desdits éléments transducteurs en fonction du point de convergence géométrique du sous-ensemble auquel a été affecté ledit élément transducteur.

12. Procédé selon la revendication 11, **caractérisé en ce qu'**on ajuste l'affectation d'au moins certains éléments transducteurs pour obtenir une répartition homogène desdits sous-ensembles d'éléments transducteurs à la surface dudit support unique.

13. Procédé selon la revendication 11 ou 12, **caractérisé en ce qu'**à l'étape a), on place autant d'éléments transducteurs de sorte que les surfaces cumulées de l'ensemble de ces éléments transducteurs représentent au moins cinquante pour cent (50%) de la surface dudit support unique.

14. Machine de traitement thérapeutique équipée d'au moins un transducteur d'ultrasons à usage médical selon l'une quelconque des revendications 1 à 9.

## Patentansprüche

1. Ultraschallwandler in Phasengitter für medizinische Zwecke, wobei der Wandler einen einzigen Träger (2) und eine Einheit von Wandlerelementen (3) aufweist, die auf der Oberfläche des Trägers (2) fest sind, wobei
- die Einheit mindestens zwei Untereinheiten von Wandlerelementen (3), wobei jede der Untereinheiten von Wandlerelementen (3) einen unterschiedlichen geometrischen Konvergenzpunkt derart aufweist, um eine Ultraschallenergie in einem unterschiedlichen Abdeckungsvolumen (4, 5) derart zu fokussieren, dass die Anordnung der Abdeckungsvolumina ein zu behandelndes Zielvolumen abdeckt, und
- Steuermittel aufweist, um die Wandlerelemente (3) unabhängig voneinander zu steuern, und wobei
- die Wandlerelemente (3) der unterschiedlichen Untereinheiten zufällig verteilt sind, indem sie auf der Oberfläche des einzigen Trägers (2) miteinander vermischt sind.

2. Wandler nach Anspruch 1, **dadurch gekennzeichnet, dass** die Wandlerelemente (3) von jeder Untereinheit auf der Oberfläche des einzigen Trägers (2) derart angeordnet sind, dass die Konvergenzpunkte dieser Untereinheiten nach mindestens einer Achse ausgerichtet sind.

3. Wandler nach Anspruch 2, **dadurch gekennzeichnet, dass** das zu behandelnde Zielvolumen entlang einer Verschiebungsachse beweglich ist, wobei die Konvergenzpunkte auf der Verschiebungsachse angeordnet sind.

4. Wandler nach Anspruch 2, **dadurch gekennzeichnet, dass** die Einheit mindestens vier Untereinheiten aufweist, wobei die Wandlerelemente (3) von jeder Untereinheit auf der Oberfläche des einzigen Trägers (2) derart angeordnet sind, dass die Konvergenzpunkte von mindestens zwei ersten Untereinheiten auf einer ersten Achse angeordnet sind und die Konvergenzpunkte von mindestens zwei zweiten Untereinheiten auf einer zweiten Achse angeordnet sind, wobei die erste und die zweite Achse nicht parallel sind.

5. Wandler nach einem der Ansprüche 1 bis 4, **dadurch gekennzeichnet, dass**, nachdem jede der Untereinheiten von Wandlerelementen eine eigene und unterschiedliche Resonanzfrequenz aufweist, ein Wandlerelement einer Untereinheit mit einem Wandlerelement von jeder anderen Untereinheit des Ultraschallwandlers im Phasengitter derart verbunden ist, dass die Wahl einer bestimmten Anregungsfrequenz, die von den Steuermitteln bereitgestellt wird, eine einzige Untereinheit von Wandlerelementen aktiviert.

6. Wandler nach einem der Ansprüche 1 bis 4, **dadurch gekennzeichnet, dass** die Steuermittel ein Haupt-Anregungssignal emittieren, das eine Überlagerung von mindestens zwei sekundären Anregungssignalen, die unterschiedliche Anregungsfrequenzen haben, aufweist, wobei jede der Untereinheiten von Wandlerelementen (3) auf eine einzige Anregungsfrequenz antwortet, wobei diese Anregungsfrequenzen für alle Untereinheiten unterschiedlich sind.

7. Wandler nach einem der Ansprüche 1 bis 6, **dadurch gekennzeichnet, dass**, nachdem die Steuermittel durch Verbindungen mit den Wandlerelementen (3) verbunden sind, mindestens bestimmte der Verbindungen die Steuermittel mit Gruppen von Wandlerelementen (3) verbinden, wobei jede Gruppe mindestens ein Wandlerelement von mindestens zwei unterschiedlichen Untereinheiten aufweist.

8. Wandler nach einem der Ansprüche 1 bis 7, **dadurch gekennzeichnet, dass** die kumulierten Oberflächen der Einheit von Wandlerelementen mindestens fünfzig Prozent (50 %) der Oberfläche des einzigen Trägers ausmachen.

9. Wandler nach einem der Ansprüche 1 bis 8, **dadurch gekennzeichnet, dass** die Geometrie des einzigen Trägers aus der Gruppe ausgewählt ist, die einen kegelstumpfförmigen, paraboloiden, zylindrischen Konus oder einen Konus von länglicher und gebogener Form aufweist.

10. Verfahren zur Herstellung eines Ultraschallwandlers nach einem der Ansprüche 1 bis 9, **dadurch gekennzeichnet, dass** es mindestens die folgenden aufeinanderfolgenden Schritte aufweist, wobei die Schritte durch mindestens eine Recheneinheit durchgeführt werden:
a) bei einem einzigen Träger (2) werden auf der Oberfläche des Trägers (2) Wandlerelemente (3) zufällig verteilt, die eine erste Untereinheit von Wandlerelementen (3) definieren, die dazu bestimmt ist, eine Ultraschallenergie in einem ersten Abdeckungsvolumen (4, 5) zu fokussieren,
b) nachdem die Wandlerelemente (3) der ersten Untereinheit fest sind, werden auf der Oberfläche des Trägers (2), der die erste Untereinheit aufweist, Wandlerelemente (3) zerstreut, die eine zweite Untereinheit von Wandlerelementen (3) definieren, die dazu bestimmt ist, eine Ultraschallenergie in einem zweiten Abdeckungsvolumen (4, 5) zu fokussieren, das sich von dem ersten Volumen unterscheidet,
c) die Position jedes der Wandlerelemente (3) dieser zweiten Untereinheit wird derart angepasst, um die Gitterkeulen zu begrenzen, die in der Brennebene auftreten können, wenn diese Untereinheiten von Wandlerelementen (3) Symmetrien aufweisen würden, wobei ein Mindestabstand d zwischen jedem Wandlerelement beibehalten wird,
d) nachdem die Wandlerelemente (3) von jeder auf diese Weise gebildeten Untereinheit fest sind, werden eventuell die Schritte b) und c) für jede neue Untereinheit von Wandlerelementen (3) wiederholt, die dazu bestimmt ist, eine Ultraschallenergie in einem Abdeckungsvolumen (4, 5) zu fokussieren, das sich von den anderen Abdeckungsvolumina unterscheidet, und
- wobei die Einheit von Wandlerelementen (3) auf der Oberfläche des einzigen Trägers (2) vermischt wird.

11. Verfahren zur Herstellung eines Ultraschallwandlers nach einem der Ansprüche 1 bis 9, **dadurch gekennzeichnet, dass** es mindestens die folgenden aufeinanderfolgenden Schritte aufweist, wobei gewisse der Schritte durch mindestens eine Recheneinheit durchgeführt werden:
a) auf der Oberfläche eines einzigen Trägers n werden Wandlerelemente angeordnet,
b) jedes auf diese Weise angeordnete Wandlerelement wird einer Untereinheit von Wandlerelementen zufällig zugeordnet, wobei die Anzahl von Untereinheiten durch die Anzahl von gewünschten geometrischen Konvergenzpunkten gegeben ist,
c) der Normalvektor von jedem der Wandlerelemente wird in Abhängigkeit von dem geometrischen Konvergenzpunkt der Untereinheit ausgerichtet, der das Wandlerelement zugeordnet worden ist.

12. Verfahren nach Anspruch 11, **dadurch gekennzeichnet, dass** die Zuordnung von mindestens gewissen Wandlerelementen angepasst wird, um eine gleichmäßige Verteilung der Untereinheiten von Wandlerelementen auf der Oberfläche des einzigen Trägers zu erhalten.

13. Verfahren nach Anspruch 11 oder 12, **dadurch gekennzeichnet, dass** in dem Schritt a) ebenso viele Wandlerelemente derart angeordnet werden, dass die kumulierten Oberflächen der Einheit von diesen Wandlerelementen mindestens fünfzig Prozent (50 %) der Oberfläche des einzigen Trägers ausmachen.

14. Maschine zur therapeutischen Behandlung, die mit mindestens einem Ultraschallwandler für medizinische Zwecke nach einem der Ansprüche 1 bis 9 ausgestattet ist.

## Claims

1. Phased-array ultrasound transducer for medical use, said transducer comprising a single support (2) and a set of transducer elements (3) which are fixed on the surface of said support (2), in which
- said set comprises at least two subsets of transducer elements (3), each of said subsets of transducer elements (3) having a distinct point of geometrical convergence so as to focus an ultrasound energy in a distinct coverage volume (4, 5) such that the arrangement of said coverage volumes covers a target volume to be treated,
- control means for controlling said transducer elements (3) independently of one another, and in which
- The transducer elements (3) of the different subsets are distributed randomly by being mixed together on the surface of the single support (2).

2. Transducer according to Claim 1, **characterized in that** the transducer elements (3) of each subset are placed on the surface of said single support (2) such that the points of convergence of these subsets are aligned on at least one axis.

3. Transducer according to Claim 2, **characterized in that**, the target volume to be treated being mobile along a displacement axis, said points of convergence are placed on said displacement axis.

4. Transducer according to Claim 2, **characterized in that**, said set comprising at least four subsets, the transducer elements (3) of each subset are placed on the surface of said single support (2) such that the points of convergence of at least two first subsets are placed on a first axis and the points of convergence of at least two second subsets are placed on a second axis, said first and second axes being non-parallel.

5. Transducer according to any one of Claims 1 to 4, **characterized in that**, each of said subsets of transducer elements having a specific and distinct resonance frequency, a transducer element of a subset is linked to a transducer element of each other subset of the phased-array ultrasound transducer such that the choice of a given excitation frequency, delivered by the control means, activates a single subset of transducer elements.

6. Transducer according to any one of Claims 1 to 4, **characterized in that** said control means emit a main excitation signal comprising a superposition of at least two secondary excitation signals having distinct excitation frequencies, each of said subsets of transducer elements (3) responding to a single excitation frequency, these excitation frequencies being different for all the subsets.

7. Transducer according to any one of Claims 1 to 6, **characterized in that**, said control means being linked to said transducer elements (3) by links, at least some of said links link said control means to groups of transducer elements (3), each group comprising at least one transducer element of at least two distinct subsets.

8. Transducer according to any one of Claims 1 to 7, **characterized in that** the aggregate surfaces of all the transducer elements represent at least fifty percent (50%) of the surface of said single support.

9. Transducer according to any one of Claims 1 to 8, **characterized in that** the geometry of said single support is chosen from the group comprising a truncated cone, paraboloid, cylindrical or even of elongate and incurved form.

10. Method for fabricating an ultrasound transducer according to any one of Claims 1 to 9, **characterized in that** it comprises at least the following successive steps, said steps being performed by at least one computation unit:
a) having a single support (2), transducer elements (3) defining a first subset of transducer elements (3) intended to focus an ultrasound energy in a first coverage volume (4, 5) are distributed randomly on the surface of said support (2),
b) the transducer elements (3) of the first subset being fixed, transducer elements (3) defining a second subset of transducer elements (3) intended to focus an ultrasound energy in a second coverage volume (4, 5) distinct from said first volume are scattered on the surface of said support (2) comprising said first subset,
c) the position of each of the transducer elements (3) of this second subset is adjusted so as to limit the array lobes which could appear in the focal plane if this subset of transducer elements (3) included symmetries, while maintaining a minimum distance d between any two transducer elements,
d) the transducer elements (3) of each duly formed subset being fixed, steps b) and c) are repeated as necessary for each new subset of transducer elements (3) intended to focus an ultrasound energy in a coverage volume (4, 5) distinct from said other coverage volumes, and
- all of the transducer elements on the surface of the single support being intermingled.

11. Method for fabricating an ultrasound transducer according to any one of Claims 1 to 9, **characterized in that** it comprises at least the following successive steps, some of said steps being performed by at least one computation unit:
a) n transducer elements are positioned on the surface of a single support,
b) each duly positioned transducer element is randomly assigned to a subset of transducer elements, the number of subsets being given by the number of desired points of geometrical convergence,
c) the normal vector of each of said transducer elements is oriented as a function of the point of geometrical convergence of the subset to which said transducer element has been assigned.

12. Method according to Claim 11, **characterized in that** the assignment of at least some transducer elements is adjusted to obtain a uniform distribution of said subsets of transducer elements on the surface of said single support.

13. Method according to Claim 11 or 12, **characterized in that**, in step a), as many transducer elements are placed such that the aggregate surfaces of all of these transducer elements represent at least fifty percent (50%) of the surface of said single support.

14. Therapeutic treatment machine equipped with at least one ultrasound transducer for medical use according to any one of Claims 1 to 9.
